# NEUE EUROPÄISCHE PATENTSCHRIFT

(11) **EP 1 575 908 B2**
(45) Veröffentlichungstag und Bekanntmachung des Hinweises auf die Entscheidung über den Einspruch: **20.01.2010**
(45) Hinweis auf die Patenterteilung: 09.08.2006
(21) Anmeldenummer: 03785813.1
(22) Anmeldetag: 13.12.2003
(51) Int. Cl.: C07C 263/20

(54) **VERFAHREN ZUR ABTRENNUNG UND REINIGUNG VON LÖSUNGSMITTEL VON EINEM REAKTIONSGEMISCH AUS EINER ISOCYANATSYNTHESE**
METHOD FOR SEPARATING OUT SOLVENT FROM A REACTION MIXTURE RESULTING FROM AN ISOCYANATE SYNTHESIS AND FOR PURIFYING THIS SOLVENT
PROCEDE DE SEPARATION ET DE PURIFICATION D'UN SOLVANT CONTENU DANS UN MELANGE REACTIONNEL RESULTANT D'UNE SYNTHESE D'ISOCYANATE

(30) Priorität: 19.12.2002 DE 10260027
(43) Veröffentlichungstag der Anmeldung: 21.09.2005
(73) Patentinhaber: BASF SE, 67056 Ludwigshafen (DE)
(72) Erfinder: SOHN, Martin, 68229 Mannheim (DE); STROEFER, Eckhard, 68163 Mannheim (DE); NEVEJANS, Filip, B-9170 St.Gillis-Waas (BE); PENZEL, Ulrich, 01945 Tettau (DE); PALLASCH, Hans-Jürgen, 67169 Kallstadt (DE); VAN DEN ABEEL, Peter, B-2930 Brasschaat (BE); DEBERDT, Filip, B-2812 Muizen (BE); JACOBS, Jan D., Baton Rouge, LA 70810 (US); MACKENROTH, Wolfgang, 67089 Bad Dürkheim (DE)
(86) Internationale Anmeldenummer: PCT/EP2003/014187
(87) Internationale Veröffentlichungsnummer: WO 2004/056761

(56) Entgegenhaltungen:
- FR-A- 1 399 472
- US-A- 3 410 888

## Beschreibung

Die Erfindung betrifft ein Verfahren zur Abtrennung des Lösungsmittels bei der Herstellung von aromatischen oder aliphatischen Isocyanaten.

Isocyanate werden, insbesondere als Ausgangsprodukte für die Herstellung von Polyurethanen, seit langem in großer Menge hergestellt. Bei den aromatischen Isocyanaten haben Methylendi(phenylisocyanat) (MDI) und dessen höhere Homologen, und Toluylendiisocyanat (TDI), bei den aliphatischen Hexamethylendiisocyanat (HDI) und Isophorondiisocyanat (IPDI) die größte technische Bedeutung.

Die kontinuierliche Herstellung von organischen Isocyanaten durch Reaktion von primären organischen Aminen mit Phosgen ist vielfach beschrieben und wird im großtechnischen Maßstab durchgeführt (s. z.B. Ullmanns Enzyklopädie der Technischen Chemie, and 7 (Polyurethane), 3. neubearbeitete Auflage, Carl Hanser Verlag, München-Wien, S. 76ff (1993)).

Die Herstellung von Isocyanaten aus den entsprechenden Aminen durch Phosgenierung erfolgt zumeist in Rührkesseln, in Rührkesselkaskaden, in mit Füllkörpern gefüllten Reaktionssäulen oder Reaktionskolonnen oder in ungefüllten Kolonnen. Oftmals ist eine Kreislauffahrweise erforderlich, um genügend Verweilzeit für einen vollständigen Umsatz bei begrenztem Reaktionsvolumen (Holdup) zu schaffen. Da die Reaktion von Amin und Phosgen in der Flüssigphase sehr schnell ist, wird für die erste Reaktionsstufe häufig ein Mischreaktor eingesetzt. Zu den bekannten Mischaggregaten gehören vor allem Düsen, wie beispielsweise Ringschlitzdüse, Ringlochdüsen, Glattstrahlmischdüsen, Fächerstrahldüsen, Winkelstrahlkammerdüsen, Dreistromdüsen, Gegenstrommischkammern, Staudüsen oder Venturimischdüsen.

Die Isocyanatsynthese erfolgt häufig in der ersten Stufe bei sehr tiefer und in der zweiten bei deutlich höherer Temperatur in einem Verweilzeitapparat (Kalt-Heiß-Phosgenierung) (W. Siefken, Liebigs Analen der Chemie 562 (1949), Seite 96). Zuerst wird bei tiefer Temperatur, insbesondere 0°C oder Raumtemperatur, maximal 60°C, eine Suspension der Zwischenprodukte Carbamoylchlorid und Aminhydrochlorid hergestellt, die dann bei höheren Temperaturen, insbesondere 100 bis 200°C, in einem Verweilzeitapparat zum Isocyanat umgesetzt wird. Solche zweistufige Verfahren werden beispielsweise in Ullmanns Enzyklopädie der Technischen Chemie, Band 7 (Polyurethane), 3. neubearbeitete Auflage, Carl Hanser Verlag, München-Wien, S. 76ff (1993), beschrieben.

Zur Erzielung hoher Ausbeuten werden die Synthesen von Isocyanaten aus Aminen und Phosgen in der Regel in organischen Lösungsmitteln durchgeführt, die sich gegenüber den Einsatzstoffen und den Endprodukten inert verhalten, In einer besonderen Ausführungsform der Isocyanatsynthese kann auch das in dem betreffenden Verfahren hergestellte Isocyanat als Lösungsmittel sowohl für das Amin als auch für das Phosgen verwendet werden.

Als inerte organische Lösungsmittel für die Herstellung von Isocyanaten werden vorzugsweise chlorierte aromatische Kohlenwasserstoffe wie Dichlorbenzol, Chlorbenzol, Trichlorbenzol oder aromatische oder aliphatische Kohlenwasserstoffe wie Toluol, Xylol, Benzol, Pentan, Hexan, Heptan, Octan, Cyclohexan, Biphenyl, Ketone wie 2-Butanon, Methylisobutylketon, Ester wie Diallylisophthalate, Ethylacetat, Butylacetat, Nitrile wie Acetonitril, oder Sulfolan u.a. verwendet.

Nach erfolgter Reaktion wird das in der Regel leichter als das Isocyanat siedende Lösungsmittel vom Isocyanat und einem eventuellen Rückstand abgetrennt und destillativ aufgearbeitet. Im Falle von Toluylendiisocyanat (TDI) erfolgt anschließend eine destillative Abtrennung des Isocyanats vom Rückstand und eine Reindestillation des Isocyanats oder eine Reinigung durch Kristallisation. Es können außerdem weitere Trennoperationen durchgeführt werden, um im Falle von TDI, und MDI das Isomeren- oder im Falle von Polymer-MDI das Oligomerengemisch in einzelne Fraktionen mit unterschiedlicher Isomeren- und Oligomerenzusammensetzng zu zerlegen. TDI liegt meist als Gemisch der beiden Isomere 2,4-TDI und 2,6-TDI vor, in der Regel als 80:20 oder 65:35 Mischung.

Das bei der Umsetzung von aliphatischen oder aromatischen Aminen mit Phosgen zu den entsprechenden Isocyanaten anfallende Gemisch aus Chlorwasserstoff und Phosgen kann ebenfalls noch mehr oder weniger große Mengen Lösungsmittel enthalten. Dieses Gemisch wird dann in der Regel in den meist gasförmig anfallenden Chlorwasserstoff und ein in der Regel flüssig anfallendes Gemisch aus Phosgen und gegebenenfalls. Lösungsmittel aufgetrennt. Das Phosgen bzw. Phosgen-Lösungsmittelgemisch wird dann in die Reaktion zurückgeführt. Es wird in der Regel ein mehr oder weniger großer Phosgenüberschuß eingesetzt, so daß das eingesetzte Phosgen trotz hoher chemischer Ausbeuten der Amin-Phosgen-Reaktion im Falle der Isocyanatproduktion nicht vollständig mit dem Amin abreagiert. Lösungsmittel in diesem Rückführphosgenstrom stört in der Regel nicht, es führt allerdings zu einer Verdünnung des Phosgenstroms.

Dagegen muß das Isocyanat vollständig aus dem Lösungsmittel, das in die Reaktion zurückgeführt wird, abgetrennt werden, um Nebenreaktionen des Isocyanats mit dem Amin auszuschließen.

In US 3410888 wird ein Verfahren zur Isolierung eines aromatischen Diisocyanats aus einer Reaktionsmischung beschrieben, wobei das Isocyanat zwei Phenylkerne besitzt und die Isocyanatgruppen an Kohlenstoffatomen unterschiedlicher Phenylkerne gebunden sind. Dies betrifft 4,4'-, 2,4'-, 2,2'-Methylendi(phenylisocyanat) (MDI) sowie Mischungen dieser Isomere oder Polymethylenpolyphenylenpolyisocyanat (PMDI). Das beanspruchte Verfahren umfaßt die Schritte erstens der Reaktion eines entsprechenden aromatischen Diamins mit Phosgen und der destillativen Abtrennung eines Teils des so hergestellten aromatischen Isocyanats bei der Lösungsmittelabtrennung, zweitens der Überführung des Destillationsrückstandes (Sumpfproduktes) in eine zweite Destillationseinrichtung, über deren innere Oberfläche der Rückstand als dünner Film verteilt ist und deren Temperatur und Druck ausreichend sind, um eine Verdampfung des Isocyanats zu bewirken, und drittens der Entnahme des Dampfes (Brüden) aus dieser zweiten Destillationseinrichtung, der im wesentlichen reich an Isocyanat ist. Der Dampf wird kondensiert und das Isocyanat gelagert. Als mögliche Destillationseinrichtung werden Kletterfilmverdampfer, Fallfilmverdampfer u.ä. genannt. Das Lösungsmittel in der Isocyanatsynthese hat üblicherweise einen niedrigeren Siedepunkt als das Isocyanat, er ist bevorzugt mindestens 30°C niedriger. Bei einer kleineren Siedepunktsdifferenz kann in der Lösungsmittelabtrennung ein Teil des hergestellten Isocyanats zusammen mit dem Lösungsmittel mitabgetrennt werden. Daran schließt sich die Destillation des als Rückstand erhaltenen Rohisocyanats im Dünnschichtverdampfer an. Die teilweise Abtrennung des Isocyanats in der Lösungsmittelabtrennung hat den Vorteil, dass unerwünschte Mittelsieder, wie gefärbte Verunreinigungen oder Komponenten, deren Siedepunkt zwischen dem des Isocyanats und dem Lösungsmittel liegt, in der Lösungsmittelabtrennung mitabgetrennt werden. Die Mischung aus dem teilweise abgetrennten Isocyanat und dem Lösungsmittel wird dann wieder als Feed der Lösungsmittelabtrennung zugeführt, oder sie wird in einer separaten Verdampfung oder fraktionierten Destillation zur Aufkonzentration des Isocyanats zugeführt. Letzteres wird dann als Feed in die Lösungsmittelabtrennung rezykliert.

Nachteilig an diesem Verfahren ist die teilweise Abtrennung des Isocyanats in der Lösungsmittelabtrennung, die eine zusätzliche Reinigung des Lösungsmittels erforderlich macht.

In US 3,544,611 wird ein Verfahren zur Herstellung von Isocyanaten durch Umsetzung von Aminen mit Phosgen in Anwesenheit von inerten Lösungsmitteln beschrieben.

Nach der Umsetzung erfolgt die Abtrennung der Lösungsmittel. Dazu wird die Mischung aus Lösungsmittel und Isocyanat zunächst in einer Kolonne mit einem geringen Druck Destilliert und das Sumpfprodukt der Isocyanat und Lösungsmittel enthält, in einer zweite Kolonne destilliert, die bei einem höheren Druck betrieben wird. Das Kopfprodukt der zweiten Kolonne wird dabei zum Verdampfen des Sumpfprodukts der ersten Kolonne eingesetzt.

Ziel der vorliegenden Erfindung ist es, ein Verfahren zur Lösungsmittelabtrennung zu entwickeln, welches eine vollständige Abtrennung des Isocyanats aus dem Lösungsmittel erlaubt und darüber hinaus Einsparungen an Energie erbringt.

Die Lösung der Aufgabe eines energiesparenden Verfahrens zur Lösungsmittelabtrennung aus einem Reaktionsaustrag der Isocyanatsynthese besteht in einem zwei- oder mehrstufigen, vorzugsweise zweistufigen destillativen Verfahren, wobei das Lösungsmittel in einem ersten Apparat, vorzugsweise einer Destillationskolonne, bei 0,1 bis 15 bar, bevorzugt 0,5 bis 3 bar, und in einem zweiten oder weiteren Apparaten, vorzugsweise ebenfalls Destillationskolonnen, bei 1 bis 900 mbar, bevorzugt 100 bis 500 mbar, abgetrennt wird, wobei die Kondensationswärme des Lösungsmitteldampfes, im folgenden auch als Brüden bezeichnet, aus dem ersten Apparat zur partiellen oder vollständigen Verdampfung von Lösungsmittel im zweiten Apparat verwendet wird.

Gegenstand der Erfindung ist demzufolge ein Verfahren nach Anspruch 1.

Die Sumpftemperatur in der ersten Kolonne beträgt je nach Druck zwischen 60°C bei 0,1 bar und 270°C bei 15 bar. Die Sumpftemperatur in der zweiten Kolonne beträgt je nach Druck zwischen 75°C bei 1 mbar und 250°C bei 900 mbar.

In der ersten Kolonne wird Lösungsmitteldampf destillativ abgetrennt. Der flüssige Anteil, der als Sumpfaustrag dieser Kolonne anfällt, wird vor oder im zweiten Apparat auf das niedrigere Druckniveau des zweiten Apparates entspannt und dem zweiten Apparat zugeführt, in welchem das restliche Lösungsmittel abgeführt wird. Die Übertragung der Energie des Brüden aus dem ersten Apparat auf die Flüssigphase des zweiten kann insbesondere unter Verwendung eines Wärmetauschers, beispielsweise eines Kreuzstromapparates, erfolgen, in dem der kondensierende Brüden die Verdampfung der flüssigen Phase aus dem zweiten Apparat der zweiten Kolonne bewirkt. Dabei können die Brüden und Flüssigphase im Gleich- oder im Gegenstrom geführt werden. Der Brüden kann im Mantel- oder im Produktraum des Wärmetauschers kondensiert werden. Es ist hierbei nicht erforderlich, einen speziellen Wärmetauscher zu verwenden Es kann ein beliebiger Apparat, der Wärmeübertragungsfläche zur Verfügung stellt, verwendet werden. Die Flüssigkeit der zweiten Kolonne kann aus dem Sumpf, von einem Boden, einem Flüssigkeitssammler oder aus dem Zulauf entnommen werden. Bevorzugt wird die Flüssigkeit von einem Boden oder Sammler unterhalb des Zulaufs der zweiten Kolonne entnommen. Beide Kolonnen sind vorzugsweise mit einem Verstärkungsteil ausgerüstet. Die zweite Kolonne kann auch mit einem Abtriebsteil ausgestattet sein.

Als Einbauten kommen die bekannten Einbauten von Destillations- und Rektifikationskolonnen zum Einsatz. Es kann beispielsweise eine Boden- oder eine Packungskolonne zum Einsatz kommen. Als Böden können beispielsweise Sieb-, Ventil-, Glocken- oder Dualflowböden und bei Packungen z.B. Blech-, Gewebe- oder Gitterpakkungen aller Art verwendet werden. Besonders vorteilhaft sind Packungen, da sie einen geringen Druckverlust aufweisen. Füllkörperschüttungen sind zwar weniger geeignet, aber nicht prinzipiell ausgeschlossen. Als Packungstypen können beispielsweise Sulzer BX, Sulzer CY, Sulzer Mellapak, Sulzer Mellapak Plus, Montz A3, Glitsch 4A, Kühni Rombopak, und andere. verwendet werden. Als Sumpfumlaufverdampfer können prinzipiell alle Arten von Verdampfertypen eingesetzt werden, wobei Fallfilmverdampfer oder Dünnschichtverdampfer besonders vorteilhaft sind, da sich mit ihnen eine produktschonende Verdampfung realisieren läßt und sie auch bei kleinen Temperaturdifferenzen zwischen heißer und kalter Seite betreibbar sind. Der Wärmeverbund kann auch in Form einer Zwischenverdampfung an der zweiten Kolonne ausgeführt werden. Bei einer Zwischenverdampfung wird die Flüssigkeit entsprechend von einem Boden oder Sammler der Kolonne entnommen und einem Wärmetauscher zugeführt. Aus energetischen Gründen kann es vorteilhaft sein, der erfindungsgemäß verwendeten Kolonne eine ein- oder mehrstufige Verdampfung vorzuschalten. Bei der Vorverdampfung wird der Flüssigkeitszulauf einem Verdampfer zugeführt und dadurch teilweise oder ganz verdampft. Der Dampf- und gegebenenfalls. verbliebene Flüssigkeitsstrom wird der Kolonne zugeführt. Sowohl die Vorverdampfung als auch die Zwischenverdampfung können ein- oder mehrstufig ausgeführt werden. Als Verdampfer können ein Durchlaufverdampfer, vorzugsweise ein Fallfilmverdampfer, Langrohrverdampfer oder Dünnschichtverdampfer verwendet werden. Der Kopfkondensator kann extern gestaltet oder in die Kolonne integriert sein. Es können Rohrbündel- wie auch Plattenapparate zum Einsatz kommen.

Das beschriebene Verfahren kann auch mehrstufig, das heißt unter Verwendung von mehr als zwei Kolonnen, ausgeführt werden bei dieser Ausführungsform wird bei jedem folgenden Apparat auf ein gegenüber dem vorherigen Apparat niedrigeres Druckniveau entspannt und ein- oder mehrmals die Energie des Brüden zur partiellen oder vollständigen Verdampfung des Flüssigphase des nachfolgenden Apparates verwendet. Ein Verfahren mit mehr als 5 Stufen ist aus Gründen der Investitionskosten nicht mehr sinnvoll, aber nicht prinzipiell ausgeschlossen.

Im erfindungsgemäßen Verfahren wird in der Lösungsmittelabtrennung kein Isocyanat zusammen mit dem Lösungsmittel entnommen, sondern das Ziel der Destillation ist eine saubere Abtrennung des Lösungsmittels unter Minimierung des Isocyanatgehalts.

Dadurch ist es möglich, eine vollständige Abtrennung des Lösungsmittels vom Reaktionsgemisch vorzunehmen, so dass es ohne weitere Aufarbeitung oder eine zusätzliche Reinigung wieder in den Herstellungsprozeß der Isocyanate zurückgeführt werden kann. Durch die Nutzung der Brüden zur Verdampfung des Reaktionsgemisches kann das Verfahren energetisch vorteilhaft betrieben werden.

Das erfindungsgemäße Verfahren kann besonders vorteilhaft zur Aufarbeitung des Lösungsmittels bei der Herstellung von TDI, MDI und HDI eingesetzt werden. Als Lösungsmittel bei der Herstellung von TDI, MDI und HDI können Chlorbenzol, Dichlorbenzol oder Mischungen der beiden oder Toluol verwendet werden.

Die Erfindung soll an dem nachstehenden Beispiel näher erläutert werden.

### Beispiel:

6,14 kg/h eines Reaktionsaustrags aus der Synthese von Toluylendiisocyanat (TDI) ausgehend von Toluylendiamin (TDA) und Phosgen wurden dem unteren Teil einer Destillationskolonne mit 50 mm Durchmesser zugeführt. Die Kolonne ist mit 6 Schüssen Gitterpakkung (Kühni Rombopak 9M, Länge eines Schusses 630 mm) gepackt. Die Sumpftemperatur betrug 150°C, der Kopfdruck 1,3 bar abs. und die Kopftemperatur 136°C. Als Sumpfumlaufverdampfer wurde ein Rohrbündelapparat mit 13 Rohren verwendet. Die Zusammensetzung des Zulaufs (Reaktionsaustrags aus der Isocyanatsynthese) war 5,1 kg/h (82 Gew.-%) Chlorbenzol, 1,0 kg/h (16 Gew.-%) TDI inklusive schwersiedender TDI-Homologen, 0,01 kg/h (0,16 Gew.-%) CCl₄, 0,01 kg/h (0,16 Gew.-%) CHCl₃, - 0,004 kg/h (0,06 Gew.-%) Chlorwasserstoff, geringe Mengen Phosgen, und chlorierte Nebenkomponenten sowie kleinere Mengen Leichtsieder. Über Kopf der ersten Kolonne wurden 2,7 kg/h gasförmig entnommen und einem Wärmetauscher (Kreuzstromapparat) zugeführt. In diesem Wärmetauscher wurde gleichzeitig der Zulauf zu einer zweiten Kolonne durch diesen Brüdenstrom vorerhitzt und partiell verdampft. Das im Wärmetauscher anfallende Kondensat des Brüden aus der ersten Kolonne wurde zum größten Teil (1,7 kg/h) als Rückführlösungsmittel in die Isocyanatsynthese zurückgeführt und zu einem kleineren Teil (1,0 kg/h) als Rücklauf auf den Kopf der ersten Kolonne gegeben. Am Sumpf der ersten Kolonne wurden 4,44 kg/h entnommen und dem genannten Wärmetauscher, der sich im Zulauf der zweiten Kolonne befand, zugeführt. Der Zulauf befand sich im Mittelteil, d.h. zwischen Abtriebs- und Verstärkungsteil der zweiten Kolonne Die zweite Kolonne hatte ebenfalls einen Durchmesser von 50 mm Durchmesser und besaß die gleiche Packung wie die erste Kolonne. Die Sumpftemperatur dieser zweiten Kolonne betrug 183°C, der Kopfdruck 120 mbar abs. und die Kopftemperatur 68°C. Als Sumpfumlaufverdampfer diente ein Rohrbündelapparat mit 13 Rohren. Am Sumpf wurden 1,0 kg/h (97,6 Gew.-%) TDI inklusive schwersiedender TDI-Homologen, und 0,01 kg/h (1 Gew.-%) chlorierte Nebenkomponenten abgezogen. Dieser Strom wurde anschließend einer TDI-Reindestillation zugeführt. Am Kopf der zweiten Kolonne fielen 3,4 kg/h (99,3 Gew.-%) Chlorbenzol, 0,01 kg/h (0,3 Gew.-%) CCl₄, 0,01 kg/h (0,3 Gew.-%) CHCl₃, 0,004 kg/h (0,1 Gew.-%) HCl, und geringe Mengen Phosgen und geringe Mengen anderer Leichtsieder an. Dieser Strom wurde kondensiert und ebenfalls als Rückführlösungsmittel in den Reaktionsteil der TDI-Synthese zurückgeführt. Ein Teil dieses Stromes wurde als Rücklauf auf den Kopf der Kolonne zurückgeführt.

## Patentansprüche

1. Verfahren zur Herstellung von Isocyanaten durch Umsetzung von Aminen mit Phosgen im Beisein von inerten organischen Lösungsmitteln in einem Reaktor und anschließende Aufarbeitung des den Reaktor verlassenden Reaktionsgemisches, **dadurch gekennzeichnet, dass** die Lösungsmittelabtrennung in einem zwei- oder mehrstufigen destillativen Verfahren erfolgt und wobei das Lösungsmittel in einer ersten Destillationskolonne bei einem Druck von 0,1 bis 15 bar und in einer zweiten oder weiteren Destillationskolonnen, bei 1 bis 900 mbar abgetrennt wird, wobei das Druckniveau in der zweiten Destillationskolonne niedriger ist als in der ersten Destillationskolonne; die Sumpftemperatur der ersten Kolonne je nach Druck zwischen 60°C bei 0,1 bar und 270°C bei 15 bar und die der zweiten Kolonne je nach Druck zwischen 75°C bei 1 mbar und 250°C bei 900 mbar liegt und die Kondensationswärme des Lösungsmitteldampfes aus der ersten Destillationskolonne zur partiellen oder vollständigen Verdampfung von Lösungsmittel in der zweiten Destillationskolonne verwendet wird, und nachfolgend eine Reindestillation des Lösungsmittels bei Drücken zwischen 0,5 bar und 3 bar durchgeführt wird.

2. Verfahren nach Anspruch 1, **dadurch gekennzeichnet, dass** der Druck im ersten Apparat 0,5 bis 3 bar beträgt.

3. Verfahren nach einem der Ansprüche 1 oder 2, **dadurch gekennzeichnet, dass** der Druck im zweiten Apparat 50 bis 500 mbar beträgt.

4. Verfahren nach einem der Ansprüche 1 bis 3, **dadurch gekennzeichnet, dass** als Apparat zur Wärmeübertragung vom Brüden des vorhergehenden Apparates auf die Flüssigphase des nachfolgenden Apparates ein Wärmetauscher verwendet wird.

5. Verfahren nach einem der Ansprüche 1 bis 4, **dadurch gekennzeichnet, dass** der Wärmeverbund durch einen Durchlaufverdampfer, vorzugsweise einen Fallfilmverdampfer, Langrohrverdampfer oder Dünnschichtverdampfer realisiert wird.

6. Verfahren nach einem der Ansprüche 1 bis 5, **dadurch gekennzeichnet, dass** als Lösungsmittel Chlorbenzol, Dichlorbenzol oder Mischungen der beiden oder Toluol verwendet wird.

7. Verfahren nach einem der Ansprüche 1 bis 6, **dadurch gekennzeichnet, dass** das Isocyanat Toluylendiisocyanat (TDI), 4,4'-, 2,4'-, 2,2'-Methylendi(phenylisocyanat) (MDI) sowie Mischungen dieser Isomere oder Polymethylenpolyphenylenpolyisocyanat (PMDI) oder Mischungen von MDI und PMDI oder Hexamethylendiisocyanat (HDI) oder Isophorondiisocyanat (IPDI) ist.

## Claims

1. A process for preparing isocyanates by reaction of amines with phosgene in the presence of inert organic solvents in a reactor and subsequent work-up of the reaction mixture leaving the reactor, wherein the solvent is separated off in a two-stage or multistage distillation process in which the solvent is separated off at a pressure of from 0.1 to 15 bar in a first distillation column and at from 1 to 900 mbar in a second distillation column or further distillation columns, with the pressure level in the second distillation column being lower than in the first distillation column; the temperature at the bottom of the first column being, depending on the pressure, in the range from 60°c at 0.1 bar to 270°C at 15 bar and the temperature at the bottom of the second column being, depending on the pressure, in the range from 75°C at 1 mbar to 250°C at 900 mbar and the heat of condensation of the solvent vapor from the first distillation column being used for partial or complete vaporization of solvent in the second distillation column, and a purification by distillation of the solvent is subsequently carried out at pressures of from 0.5 bar to 3 bar.

2. The process according to claim 1, wherein the pressure in the first apparatus is from 0.5 to 3 bar.

3. The process according to either of claims 1 and 2, wherein the pressure in the second apparatus is from 50 to 500 mbar.

4. The process according to any of claims 1 to 3, wherein a heat exchanger is used as apparatus for transfering heat from the vapor from one apparatus to the liquid phase of the following apparatus.

5. The process according to any of claims 1 to 4, wherein the thermal coupling is achieved by means of a flow-through vaporizer, preferably a falling film evaporator, long-tube evaporator or thin film evaporator.

6. The process according to any of claims 1 to 5, wherein the solvent used is chlorobenzene, dichlorobenzene or a mixture of the two or toluene.

7. The process according to any of claims 1 to 6, wherein the isocyanate is tolylene diisocyanate (TDI), methylene-4,4'-, -2,4'- or -2,2'-di(phenyl isocyanate) (MDI) or a mixture of these isomers or polymethylenepolyphenylene polyisocyanate (PMDI) or a mixture of MDI and PMDI or hexamethylene diisocyanate (HDI) or isophorone diisocyanate (IPDI).

## Revendications

1. Procédé de préparation d'isocyanates par réaction d'amines avec du phosgène en présence de solvants organiques inertes dans un réacteur et traitement ultérieur du mélange réactionnel quittant le réacteur, **caractérisé en ce que** l'isolement de solvant a lieu dans un procédé par distillation en deux ou plusieurs étapes, le solvant étant isolé dans une première colonne de distillation à une pression de 0,1 à 15 bars et dans une deuxième colonne de distillation ou des colonnes de distillation ultérieures à 1 jusqu'à 900 mbars, le niveau de pression dans la deuxième colonne de distillation étant plus bas que dans la première colonne de distillation, la température de fond de la première colonne étant, selon la pression, comprise entre 60°C à 0,1 bar et 270°C à 15 bars et celle de la deuxième colonne étant, selon la pression, comprise entre 75°C à 1 mbar et 250°C à 900 mbars, la chaleur de condensation de la vapeur de solvant provenant de la première colonne de distillation étant utilisée pour l'évaporation partielle ou totale de solvant dans la deuxième colonne de distillation, et ensuite une distillation à l'état pur du solvant étant effectuée à des pressions entre 0,5 bar et 3 bars.

2. Procédé suivant la revendication 1, **caractérisé en ce que** la pression dans le premier appareil est de 0,5 à 3 bars.

3. Procédé suivant l'une des revendications 1 ou 2, **caractérisé en ce que** la pression dans le deuxième appareil est de 50 à 500 mbars.

4. Procédé suivant l'une des revendications 1 à 3, **caractérisé en ce qu'**on utilise un échangeur de chaleur comme appareil pour le transfert de chaleur des buées de l'appareil précédent à la phase liquide de l'appareil suivant.

5. Procédé suivant l'une des revendications 1 à 4, **caractérisé en ce que** la combinaison à chaud est réalisée par un évaporateur instantané, de préférence un évaporateur pelliculaire, un évaporateur à long tube ou un évaporateur à couche mince.

6. Procédé suivant l'une des revendications 1 à 5, **caractérisé en ce que**, comme solvant, on utilise du chlorobenzène, du dichlorobenzène et des mélanges des deux ou du toluène.

7. Procédé suivant l'une des revendications 1 à 6, **caractérisé en ce que** l'isocyanate est du diisocyanate de toluylène (TDI), du di-(phénylisocyanate) de 4,4'-, 2,4'-, 2,2'-méthylène (MDI) ainsi que des mélanges de ces isomères ou du polyisocyanate de polyméthylènepolyphénylène (PMDI) ou des mélanges de MDI et de PMDI ou du diisocyanate d'hexaméthylène (HDI) ou du diisocyanate d'isophorone (IPDI).
